# EUROPÄISCHE PATENTSCHRIFT

(11) **EP 3 209 650 B1**
(45) Veröffentlichungstag und Bekanntmachung des Hinweises auf die Patenterteilung: **15.04.2020**
(21) Anmeldenummer: 15750693.2
(22) Anmeldetag: 10.08.2015
(51) Int. Cl.: C07D 317/36

(54) **VERFAHREN ZUR HERSTELLUNG VON GLYCERINSÄURECARBONAT**
PROCESS FOR THE PRODUCTION OF GLYCERIC ACID CARBONATE
PROCEDE DE PREPARATION DE CARBONATE DE L'ACIDE GLYCERIQUE

(30) Priorität: 23.10.2014 DE 102014221523
(43) Veröffentlichungstag der Anmeldung: 30.08.2017
(73) Patentinhaber: Construction Research & Technology GmbH, 83308 Trostberg (DE)
(72) Erfinder: VAUTRAVERS, Nicolas, 68159 Mannheim (DE); TELES, Joaquim Henrique, 67165 Waldsee (DE); WÖLFLE, Heimo, 83278 Traunstein (DE); DIERKER, Markus, 40593 Düsseldorf (DE); OHLMANN, Dominik, 68163 Mannheim (DE)
(74) Vertreter: BASF IP Association
(86) Internationale Anmeldenummer: PCT/EP2015/068361
(87) Internationale Veröffentlichungsnummer: WO 2016/062424

(56) Entgegenhaltungen:
- WO-A1-2013/092011
- DATABASE CASREACT [Online] CHEMICAL ABSTRACTS SERVICE, COLUMBUS, OHIO, US; 2012, XP002746142, gefunden im STN Database accession no. 158:16312 & GU, N.-Y. ET AL.: "Preparation and characterization of composite polymer electrolytes containing surface-modified nano silica", GAODENG XUEXIAO HUAXUE XUEBAO, Bd. 33, Nr. 6, 2012, Seiten 1295-1300, JILIN DAXUE, BEIJING; CN ISSN: 0251-0790
- G. Tojo; M. Fernandez: "Oxidation of Primary Alcohols to Carboxylic Acids - A Guide to Curent Common Practice", 2007, Springer-Verlag ISBN: 0-387-35431-X pages 43-60, * Seite 51, Reaktionsschema unten; Seite 57, Reaktionsschema Mitte *

## Beschreibung

Die vorliegende Erfindung betrifft ein Verfahren zur Herstellung einer Verbindung der Formel (I) mit spezifischer Bedeutung des Substituenten R₃ 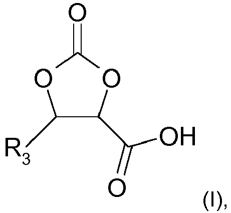 ein Gemisch sowie eine entsprechende Anwendung/Verwendung. Dokument WO 2013/092011 A1 mit dem Titel "2-OXO-1,3-DIOXOLANE-4-CARBOXAMIDES, THEIR PREPARATION AND USE" offenbart ein Verfahren zur Herstellung einer Verbindung der Formel (I), wobei R₃ H bedeutet. In WO 2013/092011 A1, Seite 10, Zeilen 6 bis 11 wird offenbart, dass die Oxidation von Glycerincarbonat (Verbindung der Formel (II), wobei R₃ H bedeutet) 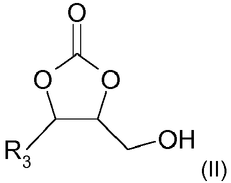 durch (i) "N-oxide-mediated oxidation" oder (ii) "aerobic oxidation" erfolgen kann. Es heißt dort unmittelbar nachfolgend: "The N-oxide-mediated oxidation may be carried out with 1,3,5-trichloroisocyanuric acid and 2,2,6,6-tetramethylpiperidin-1-oxyl (TEMPO). It may also be carried out with hydrogen peroxide as an oxidant, e.g. in the presence of a manganese salt. The aerobic oxidation uses oxygen from air or oxygen in pure form as the oxidant. It is suitably carried out in the presence of at least one transition metal salt selected from Co, Mn, Cu, Fe, and mixtures thereof, preferably Mn. It is preferably carried out in a suitable solvent or in (e.g. aqueous) acetic acid" (vgl. Seite 10, Zeilen 13 bis 20 in WO 2013/092011 A1).

Es war die Aufgabe der vorliegenden Erfindung, ein Verfahren bereitzustellen, das einen, vorzugsweise mehrere oder besonders bevorzugt sämtliche der nachfolgenden Vorteile aufweist:
- geringer operativer und technischer Aufwand,
- hoher Umsetzungsgrad der Eduktverbindungen (z.B. Umsetzung einer Verbindung der Formel (II)),
- hohe Ausbeute an Produkt (Verbindung der Formel (I)),
- einfache operative Abtrennung des Produkts vom Reaktionsmedium.

Diese Aufgabe wird gelöst durch ein erfindungsgemäßes Verfahren zur Herstellung einer Verbindung der Formel (I) 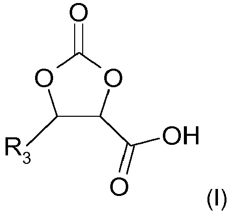 wobei R₃ ausgewählt ist aus H und geradkettigen, verzweigten oder cyclischen C₁₋₁₂-Alkylgruppen,
(a) mit folgenden Schritten:
   - Bereitstellen oder Herstellen einer Verbindung der Formel (II) 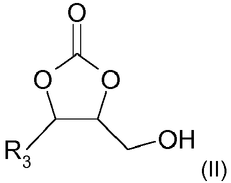 wobei R₃ die für Formel (I) gewählte Bedeutung hat
   - Platin-katalysiertes Oxidieren der Verbindung der Formel (II) mit gasförmigem Sauerstoff zu der Verbindung der Formel (I).

Die Verbindung der Formel (I) ist eine organische Säure, deren Carboxylgruppe in wässriger Lösung dissoziiert und zu einem sauren pH-Wert führt.

Der Rest R₃ ist ausgewählt aus H und geradkettigen, verzweigten oder cyclischen C₁₋₁₂-Alkylgruppen. Bevorzugt ist ein erfindungsgemäßes Verfahren (wie vorstehend beschrieben, vorzugsweise wie vorstehend als bevorzugt definiert), wobei R₃ ausgewählt ist aus der Gruppe bestehend aus H, geradkettigen C₁₋₁₂-Alkylgruppen, verzweigten C₁₋₁₂-Alkylgruppen und cyclischen C₁₋₁₂-Alkylgruppen, wobei die cyclischen C₁₋₁₂-Alkylgruppen einen oder mehrere Alkylsubstituenten an der Ringstruktur enthalten (d.h., die Ringstruktur enthält eine oder mehrere Verzweigungen) oder die cyclischen C₁₋₁₂-Alkylgruppen keine Alkylsubstituenten an der Ringstruktur enthalten (d.h., die Ringstruktur enthält keine Verzweigungen). Besonders bevorzugt ist R₃ ausgewählt aus H und geradkettigen oder verzweigten (d.h. nicht cyclischen) C₁₋₁₂-Alkylgruppen. Vorzugsweise handelt es sich bei den geradkettigen, verzweigten oder cyclischen Alkylgruppen um C₁₋₁₀, vorzugsweise C₁₋₈, besonders bevorzugt C₁₋₆-Alkylgruppen.

Je nach gewünschtem Produkt ist R₃ vorzugsweise ausgewählt aus der Gruppe bestehend aus H, Methyl, Ethyl, Propyl, Isopropyl, n-Butyl, Isobutyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, n-Hexyl, 2-Hexyl, 3-Hexyl, 2-Methylpentyl, 3-Methylpentyl, 3-Methylpentyl, 4-Methylpentyl, 2,2-Dimethylbutyl, 3,3-Dimethylbutyl, 2-Methyl-3,3-Dimethylpropyl, n-Heptyl, n-Oktyl, n-Nonyl, n-Decyl, n-Undecyl, n-Dodecyl, Cyklopentyl, Cyclohexyl und Cycloheptyl, besonders bevorzugt ausgewählt aus der Gruppe bestehend aus H, Methyl, Ethyl, Propyl, Isopropyl, n-Butyl, Isobutyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl und n-Hexyl, insbesondere ausgewählt aus der Gruppe bestehend aus H, Methyl, Ethyl, Propyl und Isopropyl.

Der einzusetzende gasförmige Sauerstoff ist vorzugsweise in Luft enthaltener gasförmiger Sauerstoff oder gasförmiger technischer (reiner) Sauerstoff oder atomarer oder molekularer Sauerstoff, der zunächst noch chemisch oder physikalisch gebunden ist und erst in freigewordenen, gasförmigen Sauerstoff überführt werden muss (Vorstufen von freiem, gasförmigem Sauerstoff). Chemisch gebundener Sauerstoff ist beispielsweise in Peroxiden gebundener Sauerstoff, der vorzugsweise durch chemische Reaktionen freigesetzt wird, sodass er als gasförmiger Sauerstoff zur Verfügung steht. Physikalisch gebundener Sauerstoff ist zum Beispiel adsorbierter gasförmiger Sauerstoff oder Sauerstoff, der in Flüssigkeiten (z.B. in Wasser) gelöst ist und beispielsweise durch Temperaturerhöhung aus der Flüssigkeit ausgetrieben werden kann, so dass er als gasförmiger Sauerstoff zur Verfügung steht. Chemisch gebundener bzw. physikalisch gebundener Sauerstoff stellt somit eine Vorstufe des in dem erfindungsgemäßen Verfahren einzusetzenden gasförmigen Sauerstoffs dar. Besonders bevorzugt ist in Luft enthaltener gasförmiger Sauerstoff oder gasförmiger reiner Sauerstoff (d.h. gasförmiger technischer Sauerstoff mit einem Sauerstoffgehalt von >99 %).

In einem erfindungsgemäßen Verfahren (wie oben beschrieben, vorzugsweise wie oben als bevorzugt definiert) können neben der als (a) gekennzeichneten Verfahrensgestaltung zusätzlich Nebenreaktionen ablaufen. Bei Durchführung des erfindungsgemäßen Verfahrens (wie vorstehend beschrieben, vorzugsweise wie vorstehend als bevorzugt definiert) kann z.B. eine Verbindung der Formel (II) in einer Nebenreaktion in eine Verbindung der Formel (III) 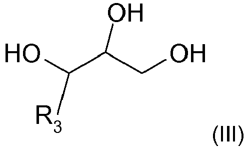 überführt werden.

Bei Durchführung des erfindungsgemäßen Verfahrens (wie vorstehend beschrieben, vorzugsweise wie vorstehend als bevorzugt definiert) kann auch alternativ oder zusätzlich zu der vorstehenden Nebenreaktion z.B. eine Verbindung der Formel (I) (das gewünschte Produkt) oder der Formel (III) in einer Nebenreaktion in eine Verbindung der Formel (IV) 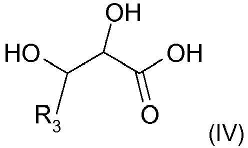 überführt werden.

Es wird nicht ausgeschlossen, dass ein Platin-katalysiertes Oxidieren der in der vorstehend genannten Nebenreaktion gebildeten Verbindung der Formel (III) mit gasförmigem Sauerstoff zu einer Verbindung der Formel (IV) führt.

Eine Verbindung der Formel (IV) kann beispielsweise aus dem Reaktionsgemisch (oder nach weiteren Prozessierungsschritten) abgetrennt werden und unter geeigneten Bedingungen in eine Verbindung der Formel (I) überführt werden (Cyclisierung z.B. mittels Phosgen oder Dimethylcarbonat; analog zu einer Herstellung der Verbindung der Formel (II)). Somit ist in manchen Fällen ein erfindungsgemäßes Verfahren (wie oben beschrieben, vorzugsweise wie oben als bevorzugt definiert) zur Herstellung einer Verbindung der Formel (I) bevorzugt 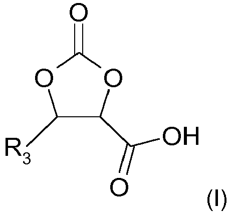 wobei R₃ ausgewählt ist aus H und geradkettigen, verzweigten oder cyclischen C₁₋₁₂-Alkylgruppen,
(a) mit folgenden Schritten:
   - Bereitstellen oder Herstellen einer Verbindung der Formel (II) 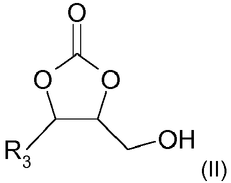 wobei R₃ die für Formel (I) gewählte Bedeutung hat
   - Platin-katalysiertes Oxidieren der Verbindung der Formel (II) mit gasförmigem Sauerstoff zu der Verbindung der Formel (I)
   und zusätzlich
(b) mit folgenden Schritten:
   - Bereitstellen oder Herstellen einer Verbindung der Formel (III) 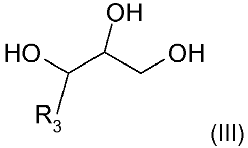 wobei R₃ die für Formel (I) gewählte Bedeutung hat
   - Platin-katalysiertes Oxidieren der Verbindung der Formel (III) mit gasförmigem Sauerstoff zu einer Verbindung der Formel (IV) 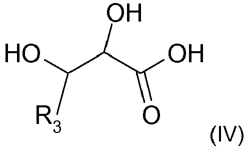 sowie optional dem Schritt:
   - Überführen der Verbindung der Formel (IV) in die Verbindung der Formel (I). Das Überführen der Verbindung der Formel (IV) in die Verbindung der Formel (I) erfolgt dabei, wie bereits oben im Text erwähnt, vorzugsweise in Gegenwart von Dimethylcarbonat oder Phosgen.

Besonders bevorzugt ist ein erfindungsgemäßes Verfahren (wie vorstehend beschrieben, vorzugsweise wie vorstehend als bevorzugt definiert), wobei zum Platin-katalysierten Oxidieren ein Katalysator eingesetzt wird, der Platin als Feststoff umfasst, bevorzugt Platin als Feststoff auf einem Trägermaterial umfasst, besonders bevorzugt Platin als Feststoff auf Kohlenstoff umfasst.
Besonders bevorzugte Trägermaterialien sind ausgewählt aus der Gruppe bestehend aus Al₂O3, Y₂O₃, LU₂O₃, ZrO₂-Y₂O₃, TiO₂, Fe₂O₃, SiO₂, Zeolite, gamma-AIO(OH), Kohlenstoff und Mischungen davon, wobei Kohlenstoff ein besonders bevorzugtes Trägermaterial ist.

Ein besonders bevorzugter Katalysator zum Platin-katalysierten Oxidieren ist ein "Platin (als Feststoff) auf Kohle"-Katalysator (auch als geträgertes Platin bekannt), wobei die Beladung des Platins auf dem Kohlenstoff vorzugsweise im Bereich von 4 w/w% Pt/C bis 12 w/w% Pt/C liegt, besonders vorzugsweise im Bereich von 5 w/w% Pt/C bis 10 w/w% Pt/C liegt. Eigene Untersuchungen haben gezeigt, dass bei einer Beladung kleiner 4 w/w% Pt/C in einigen Fällen eine unerwünscht hohe CO₂-Bildung (Decarboxylierung) beobachtet wurde.

Vorzugsweise beträgt die Gesamtstoffmenge des geträgerten Platins in einem erfindungsgemäßen Verfahren (wie vorstehend beschrieben, vorzugsweise wie vorstehend als bevorzugt definiert) 2,5 bis 10 mol%, vorzugsweise 4 bis 8 mol%, besonders bevorzugt 4 bis 6 mol%, jeweils bezogen auf die Gesamtstoffmenge der Verbindung der Formel (II).

Besonders bevorzugt ist ein erfindungsgemäßes Verfahren (wie vorstehend beschrieben, vorzugsweise wie vorstehend als bevorzugt definiert), wobei zum Platin-katalysierten Oxidieren ein "Platin (als Feststoff) auf Kohle"-Katalysator zum Oxidieren mit gasförmigem Sauerstoff eingesetzt wird, wobei
- die Beladung des Platins auf dem Kohlenstoff im Bereich von 4 w/w% Pt/C bis 12 w/w% Pt/C liegt, vorzugsweise im Bereich von 5 w/w% Pt/C bis 10 w/w% Pt/C liegt
   und
- die Gesamtstoffmenge des geträgerten Platins (wie vorstehend beschrieben, vorzugsweise wie vorstehend als bevorzugt definiert) 2,5 bis 10 mol%, vorzugsweise 4 bis 8 mol%, besonders bevorzugt 4 bis 6 mol% beträgt, jeweils bezogen auf die Gesamtstoffmenge der Verbindung der Formel (II).

Eigene Untersuchungen haben ergeben, dass regelmäßig dann gute Ergebnisse (akzeptable Ausbeuten und akzeptable Umsätze) erzielt werden, wenn ein definiertes Verhältnis von Platin zu der Gesamtmenge der Verbindung der Formel (II) besteht. Bevorzugt ist daher ein erfindungsgemäßes Verfahren (wie vorstehend beschrieben, vorzugsweise wie vorstehend als bevorzugt definiert), wobei zu Beginn des Schrittes des Platin-katalysierten Oxidierens das molare Verhältnis des eingesetzten Platins zu der Verbindung der Formel (II) größer ist als 2 : 100, vorzugsweise größer ist als 3 : 100.

Besonders bevorzugt ist ein erfindungsgemäßes Verfahren (wie vorstehend beschrieben, vorzugsweise wie vorstehend als bevorzugt definiert), wobei R₃ Wasserstoff bedeutet. In diesem Fall handelt es sich bei der Verbindung der Formel (I) um Glycerinsäurecarbonat, bei der Verbindung der Formel (II) um Glycerincarbonat (bei der Verbindung der Formel (III) um Glycerin und bei der Verbindung der Formel (IV) um Glycerinsäure). Das vorstehend und nachfolgend im Text zu bevorzugten Ausgestaltungen des erfindungsgemäßen Verfahrens Gesagte gilt insbesondere für ein bevorzugtes erfindungsgemäßes Verfahren, wobei R₃ H bedeutet.

Besonders bevorzugt ist ein erfindungsgemäßes Verfahren (wie vorstehend beschrieben, vorzugsweise wie vorstehend als bevorzugt definiert), wobei der Schritt des Platin-katalysierten Oxidierens in wässrigem Medium erfolgt. Ein wässriges Medium hat sich in der Praxis als vorteilhaft erwiesen, da die Verbindungen der Formeln (I) und (II) in einem wässrigen Medium regelmäßig eine für die vorliegenden Zwecke ausreichende Stabilität besitzen (zum Beispiel Hydrolysestabilität) bzw. die Verbindung der Formel (II) eine ausreichend gute Reaktivität besitzt. Außerdem ist ein wässriges Medium einfach bereitzustellen und nach Abschluss des erfindungsgemäßen Verfahrens vergleichsweise einfach aufzuarbeiten, um die Verbindung der Formel (I) zu isolieren.

Bevorzugt ist ein erfindungsgemäßes Verfahren (wie vorstehend beschrieben, vorzugsweise wie vorstehend als bevorzugt definiert), wobei der Schritt des Platin-katalysierten Oxidierens zumindest zeitweilig, vorzugsweise zeitlich überwiegend, bei einer Temperatur im Bereich von 50 bis 90°C, vorzugsweise im Bereich von 50 bis 82°C, besonders bevorzugt in einem Bereich von 68 bis 82°C erfolgt. Besonders bevorzugt erfolgt der Schritt des Platin-katalysierten Oxidierens bei einer Temperatur im Bereich von 50 bis 90°C, vorzugsweise im Bereich von 50 bis 82°C, besonders bevorzugt in einem Bereich von 68 bis 82°C.

Temperaturen im Bereich von 50 bis 90°C, vorzugsweise im Bereich von 50 bis 82°C, besonders bevorzugt im Bereich von 68 bis 82°C, sind besonders vorteilhaft, da solche Temperaturen bei normalem Atmosphärendruck erreicht werden können. Dies ermöglicht eine vergleichsweise einfache Verfahrensgestaltung. Temperaturen <50°C führen zu einem sehr langsamen Reaktionsverlauf, der insbesondere unwirtschaftlich ist. Temperaturen größer 82°C führen zu einer zunehmenden Zersetzung (CO₂-Bildung, Hydrolyse) der Edukte bzw. Produkte und damit zu einer sich verschlechternden Ausbeute. Dieser Effekt ist besonders ausgeprägt, wenn die Temperaturen 90°C übersteigen.

Wie bereits oben erwähnt, besitzen die Verbindungen der Formeln (I) und (II) in einem wässrigen Medium eine für die vorliegenden Zwecke ausreichende Hydrolysestabilität. Diese Hydrolysestabilität ist nicht nur von dem oder den eingesetzten Lösungsmittel(n) (vorzugsweise Wasser) abhängig, sondern zusätzlich vom pH-Wert des entsprechenden wässrigen Mediums (insbesondere bei Einsatz von Wasser als alleiniges Lösungsmittel). Wie ebenfalls bereits oben ausgeführt, handelt es sich bei der hergestellten Verbindung der Formel (I) um eine organische Säure, deren Dissoziation der Carboxylgruppe in Gegenwart von Wasser zu einer Verringerung des pH-Wertes in dem wässrigen Medium bereits während des Reaktionsverlaufes führt. pH-Werte größer 6, insbesondere >7 wirken sich nachteilig auf die Stabilität von Glycerincarbonat (Verbindung der Formel (II), wobei R₃ H beutet) und Glycerinsäurecarbonat (Verbindung der Formel (I), wobei R₃ H beutet) aus. Die Hydrolyseanfälligkeit steigt mit größer werdendem pH-Wert. Ein vergleichsweise geringer pH-Wert (kleiner 6) stabilisiert hingegen die Verbindungen der Formeln (I) und (II), wobei jeweils R₃ vorzugsweise H bedeutet.

Besonders bevorzugt ist daher ein erfindungsgemäßes Verfahren (wie vorstehend beschrieben, vorzugsweise wie vorstehend als bevorzugt definiert), wobei zu Beginn des Schrittes des Platin-katalysierten Oxidierens im wässrigen Medium ein pH von 7 oder <7 vorliegt, vorzugsweise ein pH in einem Bereich von 4 bis 7 vorliegt, vorzugsweise ein pH in einem Bereich von 4 bis 6 vorliegt.

In einem bevorzugten erfindungsgemäßen Verfahren wird der Schritt des Platin-katalysierten Oxidierens zumindest zeitweilig, vorzugsweise zeitlich überwiegend, bei einer Temperatur im Bereich von 50 bis 90°C, vorzugsweise im Bereich von 50 bis 82°C, besonders bevorzugt in einem Bereich von 68 bis 82°C durchgeführt. In diesen bevorzugten Verfahrensgestaltungen liegt der pH-Wert im wässrigen Medium zu Beginn der Oxidation vorzugsweise im Bereich von 4 bis 7, vorzugsweise im Bereich von 4 bis 6. Bei einer Temperatur im Bereich von 50 bis 90 °C, vorzugsweise im Bereich von 50 bis 82 °C, besonders bevorzugt im Bereich von 68 bis 82 °C, und einem pH-Wert im Bereich von 4 bis 7, vorzugsweise im Bereich von 4 bis 6 (jeweils bezogen auf den Beginn der Oxidation), zeigt die Verbindung der Formel (II) noch immer eine ausreichend hohe Oxidierbarkeit sowie Stabilität gegen unerwünschte Nebenreaktionen und kann somit gut zu einer Verbindung der Formel (I) umgesetzt werden.

Es hat sich in eigenen Untersuchungen herausgestellt, dass es regelmäßig von Vorteil ist, das erfindungsgemäße Verfahren so durchzuführen, dass der pH-Wert im wässrigen Medium die Folge der Dissoziation der Verbindung der Formel (I) (und ggf. zusätzlich der Formel (IV)) ist. Wie bereits oben ausgeführt, führt insbesondere die Anwesenheit der Verbindung der Formel (I) im wässrigen Medium zu einem Absinken des pH-Wertes, bis (zumindest annähernd) ein pH-Wert erreicht ist, der dem Eigen-pH-Wert dieser Verbindung (insbesondere der Verbindung der Formel (I), wobei R₃ vorzugsweise H bedeutet) im wässrigen Medium entspricht. Der Eigen-pH-Wert einer Verbindung ist der pH-Wert, der bei einer gegebenen Konzentration dieser Verbindung und aufgrund des zu dieser Verbindung gehörenden pKs-Wertes in einem wässrigen Medium vorliegt. Bevorzugt ist ein erfindungsgemäßes Verfahren (wie vorstehend definiert, vorzugsweise wie vorstehend als bevorzugt definiert), wobei der Schritt des Platin-katalysierten Oxidierens so durchgeführt wird, dass mit Bildung der Verbindung der Formel (I) der pH im wässrigen Medium fällt, vorzugsweise zumindest um 2 pH-Einheiten, besonders bevorzugt zumindest um 3 pH-Einheiten, weiter bevorzugt zumindest um 4 pH-Einheiten, insbesondere bevorzugt um 6 pH-Einheiten.

Ein um 2,5 pH-Einheiten fallender pH-Wert liegt beispielsweise vor, wenn, ausgehend von einem Start-pH-Wert von beispielsweise 4,5, der pH-Wert des wässrigen Mediums während der Oxidation auf 2 fällt (vgl. die Beispiele weiter unten im Text). Besonders bevorzugt wird der Schritt des Platin-katalysierten Oxidierens so durchgeführt, dass mit Bildung der Verbindung der Formel (I) (wobei R₃ vorzugsweise H bedeutet) der pH-Wert im wässrigen Medium im Bereich von 2 bis 0, vorzugsweise im Bereich von 1,4 bis 0,2 liegt.

Es hat sich in eigenen Untersuchungen überraschend gezeigt, dass der in dem erfindungsgemäßen Verfahren (wie vorstehend definiert, vorzugsweise wie vorstehend als bevorzugt definiert) bevorzugt eingesetzte geträgerte Platin-Katalysator (zu bevorzugten Katalysatoren siehe weiter oben im Text) in einem wässrigen Medium mit den zuvor beschriebenen pH-Werten eine noch immer ausreichende Aktivität besitzt. Verbindungen der Formeln (I) und (II), wobei R₃ vorzugsweise H bedeutet, sind unter diesen Bedingungen für die vorliegenden Zwecke vor Hydrolyse weitestgehend geschützt. Wie bereits oben erwähnt, war es eine Aufgabe der vorliegenden Erfindung, ein Verfahren bereitzustellen, welches einfach und ohne größeren operativen Aufwand durchgeführt werden kann. Da sich überraschenderweise gezeigt hat, dass der bevorzugt einzusetzende geträgerte Platin-Katalysator bei den zuvor genannten pH-Werten eine immer noch ausreichende Aktivität besitzt, ist eine fortlaufende Bestimmung und ggf. Anpassung des pH-Wertes des wässrigen Mediums in dem erfindungsgemäßen Verfahren (wie vorstehend definiert, vorzugsweise wie vorstehend als bevorzugt definiert) nicht notwendig. Üblicherweise wird der pH-Wert bei weniger sauren Bedingungen durch Zugabe von Basen in einem gewissen pH-Bereich gehalten (z.B. in Heyns-Oxidationen). In dem erfindungsgemäßen Verfahren müssen jedoch keine Maßnahmen ergriffen werden, um dem sich einstellenden Eigen-pH-Wert im wässrigen Medium durch kontinuierliche oder schrittweise Zugabe einer Base entgegenzuwirken. Es ist außerdem nicht notwendig, ein wässriges Medium mit einem Puffersystem zu verwenden, um der während der Reaktion durch Dissoziation von als Produkt gebildeter Säure auftretenden pH-Absenkung entgegenzuwirken. Dies vereinfacht die Verfahrensdurchführung und reduziert die für die Durchführung des Verfahrens notwendigen Kosten. Besonders bevorzugt ist somit ein erfindungsgemäßes Verfahren (wie vorstehend beschrieben, vorzugsweise wie vorstehend als bevorzugt definiert), wobei der Schritt des Platin-katalysierten Oxidierens ohne Zugabe von Base oder Puffer erfolgt.

Es hat sich in eigenen Untersuchungen gezeigt, dass regelmäßig eine besonders gute Ausbeute der Verbindung der Formel (I) erreicht wird, obwohl der pH-Wert im wässrigen Medium gering ist (siehe die vorstehenden Ausführungen). In manchen Fällen ist ein erfindungsgemäßes Verfahren (wie vorstehend beschrieben, vorzugsweise wie vorstehend als bevorzugt definiert) bevorzugt, wobei der Schritt des Platin-katalysierten Oxidierens nicht beendet wird, bevor der pH (von höheren pH-Werten kommend) den Wert 2, vorzugsweise den Wert 1, besonders bevorzugt den Wert 0,5 unterschreitet, wobei zu Beginn des Schrittes des Platin-katalysierten Oxidierens im wässrigen Medium ein pH-Wert von 7 oder kleiner 7 vorliegt, vorzugsweise ein pH in einem Bereich von 4 bis 7 vorliegt, vorzugsweise ein pH in einem Bereich von 4 bis 6 vorliegt
oder
wobei der Schritt des Platin-katalysierten Oxidierens zumindest solange durchgeführt wird, bis (von höheren pH-Werten kommend) ein pH-Wert von 2, vorzugsweise 1, besonders bevorzugt 0,5 unterschritten ist, wobei zu Beginn des Schrittes des Platin-katalysierten Oxidierens im wässrigen Medium ein pH-Wert von 7 oder kleiner 7 vorliegt, vorzugsweise ein pH in einem Bereich von 4 bis 7 vorliegt, vorzugsweise ein pH in einem Bereich von 4 bis 6 vorliegt.

Es ist somit besonders vorteilhaft, das erfindungsgemäße Verfahren (wie vorstehend beschrieben, vorzugsweise wie vorstehend als bevorzugt definiert) vorzugsweise so durchzuführen, dass mindestens ein Ziel-pH-Wert erreicht wird, ehe die Reaktion beendet wird (ausgehend von einem bestimmten, höher liegenden Start-pH-Wert). Ein solches Vorgehen ist besonders dann vorteilhaft, wenn das erfindungsgemäße Verfahren als Batch-Verfahren durchgeführt wird. Vorzugsweise wird der Schritt des Platin-katalysierten Oxidierens nicht beendet, bevor der pH (von höheren pH-Werten kommend) den Wert 2, vorzugsweise den Wert 1, besonders bevorzugt den Wert 0,5 unterschreitet (wobei zu Beginn des Schrittes des Platin-katalysierten Oxidierens im wässrigen Medium ein pH-Wert von 7 oder kleiner 7 vorliegt, vorzugsweise ein pH in einem Bereich von 4 bis 7 vorliegt, vorzugsweise ein pH in einem Bereich von 4 bis 6 vorliegt), oder wobei der Schritt des Platin-katalysierten Oxidierens zumindest solange durchgeführt wird, bis (von höheren pH-Werten kommend) ein pH-Wert von 2, vorzugsweise 1, besonders bevorzugt 0,5 unterschritten ist (wobei zu Beginn des Schrittes des Platin-katalysierten Oxidierens im wässrigen Medium ein pH-Wert von 7 oder <7 vorliegt, vorzugsweise ein pH in einem Bereich von 4 bis 7 vorliegt, vorzugsweise ein pH in einem Bereich von 4 bis 6 vorliegt), wobei der Schritt des Platin-katalysierten Oxidierens (jeweils bezogen auf die zwei zuvor genannten Alternativen) zumindest zeitweilig, vorzugsweise zeitlich überwiegend, bei einer Temperatur im Bereich von 50 bis 90°C, vorzugsweise im Bereich von 50 bis 82°C, besonders bevorzugt in einem Bereich von 68 bis 82°C erfolgt.

Besonders bevorzugt ist ein erfindungsgemäßes Verfahren (wie vorstehend beschrieben, vorzugsweise wie vorstehend als bevorzugt definiert), wobei die Ausbeute der Verbindung der Formel (I) 90% oder höher beträgt, nachdem der Schritt des Platin-katalysierten Oxidierens beendet ist und der pH des wässrigen Mediums vorzugsweise den Wert 0,5 unterschritten hat (vgl. die Beispiele unten im Text). Besonders bevorzugt ist ein erfindungsgemäßes Verfahren (wie vorstehend beschrieben, vorzugsweise wie vorstehend als bevorzugt definiert), wobei der Schritt des Platin-katalysierten Oxidierens nicht beendet wird, bevor der pH-Wert des wässrigen Mediums den Eigen-pH-Wert erreicht hat (bzw. der Schritt des Platin-katalysierten Oxidierens solange durchgeführt wird, bis der pH-Wert des wässrigen Mediums dem Eigen-pH-Wert der Verbindung der Formel (I) annähernd entspricht), wobei vorzugsweise der Schritt des Platin-katalysierten Oxidierens zumindest zeitweilig, vorzugsweise zeitlich überwiegend, bei einer Temperatur im Bereich von 50 bis 90°C, vorzugsweise im Bereich von 50 bis 82°C, besonders bevorzugt in einem Bereich von 68 bis 82°C erfolgt.

Wie bereits oben im Text ausgeführt, erfolgt das Platin-katalysierte Oxidieren mit gasförmigem Sauerstoff. Der Sauerstoff kann dabei auf verschiedene Weise zur Oxidation der Verbindung der Formel (II) (ggf. auch zur Oxidation einer Verbindung der Formel (III)) herangeführt werden. Besonders bevorzugt ist ein erfindungsgemäßes Verfahren (wie vorstehend beschrieben, vorzugsweise wie vorstehend als bevorzugt definiert), wobei der Schritt des Platin-katalysierten Oxidierens so durchgeführt wird, dass der gasförmige Sauerstoff in das wässrige Medium eingeleitet wird. Besonders bevorzugt ist ein erfindungsgemäßes Verfahren (wie vorstehend beschrieben, vorzugsweise wie vorstehend als bevorzugt definiert), wobei der gasförmige Sauerstoff in Form von gasförmiger Luft oder reinem gasförmigen Sauerstoff (z.B. technischer Sauerstoff mit einem Sauerstoffgehalt größer 99%) in das wässrige Medium eingeleitet wird. Vorzugsweise wird das eingeleitete Gas (z.B. gasförmige Luft oder reiner gasförmiger Sauerstoff) so in das wässrige Medium eingeleitet, dass das eingeleitete Gas das wässrige Medium durchströmt und dabei vorzugsweise die Kontaktzeit mit dem wässrigen Medium maximal ist.

Bevorzugt ist ein erfindungsgemäßes Verfahren (wie vorstehend beschrieben, vorzugsweise wie vorstehend als bevorzugt definiert), wobei der Schritt des Platin-katalysierten Oxidierens bei Atmosphärendruck durchgeführt wird. In Abhängigkeit von anderen Anwendungen kann es bevorzugt sein, das erfindungsgemäße Verfahren (wie vorstehend beschrieben, vorzugsweise wie vorstehend als bevorzugt definiert) so durchzuführen, dass der Schritt des Platin-katalysierten Oxidierens bei einem Druck in einem Bereich von 1 bis 50 bar, vorzugsweise in einem Bereich von 1 bis 10 bar, besonders bevorzugt in einem Bereich von 1 bis 2 bar, durchgeführt wird.

Besonders bevorzugt ist ein erfindungsgemäßes Verfahren (wie vorstehend beschrieben, vorzugsweise wie vorstehend als bevorzugt definiert), wobei die Verbindungen mit den Formeln (I) bis (IV) (insbesondere die Verbindungen der Formeln (I) und (II)) in einem wässrigen Medium (vorzugsweise einem wässrigen Medium, wie vorstehend beschrieben, vorzugsweise wie vorstehend als bevorzugt definiert) gelöst vorliegen (wässrige Lösung). Dies ist insbesondere dann von Vorteil, wenn für das Platin-katalysierte Oxidieren ein bevorzugter geträgerter Platin-Katalysator eingesetzt wird (zu bevorzugten Katalysatoren siehe weiter oben im Text). In diesem Fall kann der heterogene Platin-Katalysator durch einfache mechanische Trennverfahren (z.B. mittels Filtration, Zentrifugation und/oder Sedimentation) von der wässrigen Lösung abgetrennt werden. Erfolgt eine vollständige bzw. fast vollständige Umsetzung einer Verbindung der Formel (II) zu einer Verbindung der Formel (I), so enthält das vom Katalysator abgetrennte Reaktionsmedium (z.B. das Filtrat) ausschließlich oder fast ausschließlich das gewünschte Reaktionsprodukt der Formel (I). Unter diesen Bedingungen kann das Produkt der Formel (I) auf einfache Art und Weise und ohne großen operativen, technischen Aufwand gewonnen werden. In einem anschließenden Schritt wird das Lösungsmittel Wasser vorzugsweise abgezogen (verdampft), vorzugsweise unter Vakuum (z.B. in einem Rotationsverdampfer).

Die vorliegende Erfindung betrifft auch die Anwendung (bzw. die Verwendung) der Heyns-Oxidation zur Herstellung der Verbindung der Formel (I). Erfindungsgemäß betrifft die vorliegende Erfindung somit die Anwendung (bzw. die Verwendung) der Heyns-Oxidation
(a) zur Oxidation einer Verbindung der Formel (II) 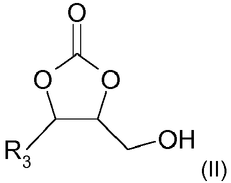
   wobei R₃ ausgewählt ist aus H und geradkettigen, verzweigten oder cyclischen C₁₋₁₂-Alkylgruppen,
   wobei die Heyns-Oxidation ohne Zugabe von Base oder Puffer erfolgt.
Das vorstehend zum erfindungsgemäßen Verfahren Gesagte gilt für die erfindungsgemäße Anwendung (bzw. Verwendung) entsprechend.

Wie bereits oben im Text erwähnt (und ohne an diese Annahme gebunden zu sein), ist es in dem erfindungsgemäßen Verfahren nicht ausgeschlossen, dass eine Verbindung der Formel (III) (die in dem bevorzugten wässrigen Medium des erfindungsgemäßen Verfahrens vorliegen kann) mittels des Schrittes des Platin-katalysierten Oxidierens in eine Verbindung der Formel (IV) überführt wird. In diesem Fall ist eine erfindungsgemäße Anwendung (bzw. Verwendung) der Heyns-Oxidation bevorzugt
(a) zur Oxidation einer Verbindung der Formel (II) 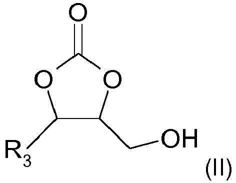 wobei R₃ ausgewählt ist aus H und geradkettigen, verzweigten oder cyclischen C₁₋₁₂-Alkylgruppen,
   und zusätzlich
(b) zur Oxidation einer Verbindung der Formel (III) 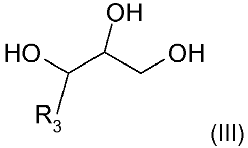
   wobei R₃ die für Formel (II) gewählte Bedeutung hat,
   wobei die Heyns-Oxidation jeweils ohne Zugabe von Base oder Puffer erfolgt.
Die "Heyns-Oxidation" bezeichnet die Oxidation von organischen primären Alkoholen zu organischen Säuren in Gegenwart von Sauerstoff und Platin in wässrigen Medien, besonders bei einem basischen pH-Wert. Details zur Heyns-Reaktion können dem Fachbuch "Oxidation of Primary Alcohols to Carboxylic Acids, A Guide to Current Common Practice" von Gabriel Tojo und Marcos Fernändez auf den Seiten 43 bis 60 entnommen werden.

Die Anwendung (bzw. Verwendung) der "Heyns-Oxidation" führt somit zu einem Verfahren (vorzugsweise zu einem bevorzugten erfindungsgemäßen Verfahren, sofern bevorzugte Temperaturen und/oder pH-Werte vorliegen), bei dem die primäre Hydroxylgruppe einer Verbindung der Formel (II) (und ggf. einer Verbindung der Formel (III)) mittels Oxidation in Gegenwart von gasförmigem Sauerstoff in eine Carboxylgruppe überführt wird (es resultiert eine Verbindung der Formeln (I) (und ggf. eine Verbindung der Formel (IV)). Die dabei ablaufende Oxidationsreaktion umfasst die Oxidation der Hydroxylgruppe zu einer Aldehydgruppe sowie die anschließende Oxidation der Aldehydgruppe zu einer Carboxylgruppe. Findet der Schritt der Oxidation unter wasserfreien Bedingungen statt, erfolgt keine vollständige Oxidation zur Carboxylgruppe, sondern die primäre Hydroxylgruppe wird nur bis zur Aldehydgruppe oxidiert.

Insbesondere bevorzugt ist die Anwendung (bzw. Verwendung) der Heyns-Oxidation (wie vorstehend beschrieben, vorzugsweise wie vorstehend als bevorzugt definiert), wobei der Schritt der Oxidation zumindest zeitweilig, vorzugsweise zeitlich überwiegend, bei einer Temperatur im Bereich von 50 bis 90°C, vorzugsweise im Bereich von 50 bis 82 °C, besonders bevorzugt im Bereich von 68 bis 82 °C, durchgeführt wird.

Besonders bevorzugt ist die erfindungsgemäße Anwendung (bzw. Verwendung) der Heyns-Oxidation (wie vorstehend beschrieben, vorzugsweise wie vorstehend als bevorzugt definiert), wobei (unabhängig von der zuvor genannten Temperatur oder zusätzlich zu der zuvor genannten Temperatur) der Schritt der Oxidation nicht beendet wird, bevor der pH (von höheren pH-Werten kommend) den Wert 2, vorzugsweise den Wert 1, besonders bevorzugt den Wert 0,5 unterschreitet, wobei zu Beginn des Schrittes des Platin-katalysierten Oxidierens im wässrigen Medium ein pH-Wert von 7 oder kleiner 7 vorliegt, vorzugsweise ein pH in einem Bereich von 4 bis 7 vorliegt, vorzugsweise ein pH in einem Bereich von 4 bis 6 vorliegt oder
wobei der Schritt des Platin-katalysierten Oxidierens zumindest solange durchgeführt wird, bis (von höheren pH-Werten kommend) ein pH-Wert von 2, vorzugsweise 1, besonders bevorzugt 0,5 unterschritten ist, wobei zu Beginn des Schrittes des Platin-katalysierten Oxidierens im wässrigen Medium ein pH-Wert von 7 oder <7 vorliegt, vorzugsweise ein pH in einem Bereich von 4 bis 7 vorliegt, vorzugsweise ein pH in einem Bereich von 4 bis 6 vorliegt.

Die vorliegende Erfindung betrifft auch ein Gemisch umfassend
- einen Katalysator, der Platin als Feststoff umfasst, bevorzugt Platin als Feststoff auf einem Trägermaterial umfasst, besonders bevorzugt Platin als Feststoff auf Kohlenstoff umfasst,
   sowie
- eine oder mehrere Verbindungen ausgewählt aus der Gruppe bestehend aus
   (a) Verbindungen der Formel (II) 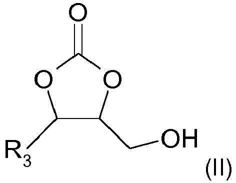 wobei R₃ ausgewählt ist aus H und geradkettigen, verzweigten oder cyclischen
      C₁₋₁₂-Alkylgruppen
      und optional eine oder mehrere Verbindungen ausgewählt aus der Gruppe bestehend aus
   (b) Verbindungen der Formel (III) 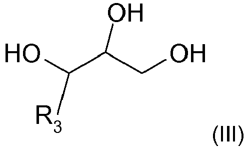 wobei R₃ ausgewählt ist aus H und geradkettigen, verzweigten oder cyclischen C₁₋₁₂-Alkylgruppen.
Vorzugsweise umfasst das erfindungsgemäße Gemisch (wie vorstehend beschrieben) sowohl eine oder mehrere (vorzugsweise eine, besonders bevorzugt eine Verbindung der Formel (II), wobei R₃ H bedeutet) Verbindungen der Formel (II) als auch eine oder mehrere (vorzugsweise eine, besonders bevorzugt eine Verbindung der Formel (III), wobei R₃ H bedeutet) Verbindungen der Formel (III), wobei der Stoffmengenanteil der Verbindung der Formel (II) vorzugsweise 95% oder größer 95% ist, vorzugsweise 97% oder größer 97% ist, bezogen auf die Gesamtstoffmenge an Verbindungen der Formeln (II) und (III).
Das zuvor zu dem erfindungsgemäßen Verfahren Gesagte bzw. das zuvor zu der erfindungsgemäßen Anwendung der Heyns-Oxidation Gesagte gilt für das erfindungsgemäße Gemisch entsprechend.

Das vorstehend beschriebene erfindungsgemäße Gemisch wird vorzugsweise zum Startzeitpunkt in einem erfindungsgemäßen Verfahren (wie vorstehend beschrieben, vorzugsweise wie vorstehend als bevorzugt definiert) oder vorzugsweise zum Startzeitpunkt bei der erfindungsgemäßen Anwendung (bzw. Verwendung) der Heyns-Oxidation (wie vorstehend beschrieben, vorzugsweise wie vorstehend als bevorzugt definiert) eingesetzt (z.B. in einer Verfahrensgestaltung, die als batch-Verfahren bekannt ist).
Wie bereits oben im Text ausgeführt, treten in einem erfindungsgemäßen Verfahren regelmäßig Nebenreaktionen auf (zu spezifischen Nebenreaktionen siehe weiter oben im Text).

In einigen Fällen ist ein erfindungsgemäßes Gemisch (wie vorstehend beschrieben, vorzugsweise wie vorstehend als bevorzugt definiert) bevorzugt, umfassend
- einen Katalysator, der Platin als Feststoff umfasst, bevorzugt Platin als Feststoff auf einem Trägermaterial umfasst, besonders bevorzugt Platin als Feststoff auf Kohlenstoff umfasst,
- eine oder mehrere (vorzugsweise eine) Verbindung der Formel (II) 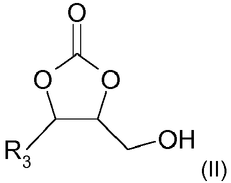 wobei R₃ ausgewählt ist aus H und geradkettigen, verzweigten oder cyclischen
   C₁₋₁₂-Alkylgruppen sowie
   (a) eine oder mehrere (vorzugsweise eine) Verbindungen der Formel (I) 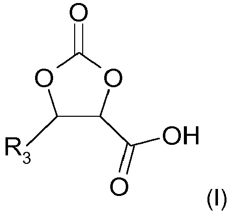 wobei R₃ ausgewählt ist aus H und geradkettigen, verzweigten oder cyclischen
      C₁₋₁₂-Alkylgruppen
      sowie vorzugsweise
   (b) eine oder mehrere (vorzugsweise eine) Verbindungen der Formel (III) 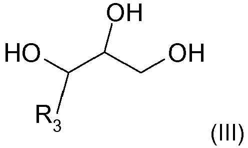 wobei R₃ ausgewählt ist aus H und geradkettigen, verzweigten oder cyclischen
      C₁₋₁₂-Alkylgruppen
      und vorzugsweise
   (c) eine oder mehrere (vorzugsweise eine) Verbindungen der Formel (IV) 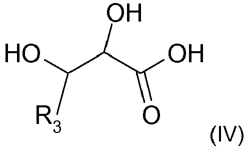 wobei R₃ ausgewählt ist aus H und geradkettigen, verzweigten oder cyclischen C₁₋₁₂-Alkylgruppen.

Das vorstehend beschriebene, bevorzugte, erfindungsgemäße Gemisch wird vorzugsweise in einem erfindungsgemäßen Verfahren (wie vorstehend beschrieben, vorzugsweise wie vorstehend als bevorzugt definiert) oder vorzugsweise bei der erfindungsgemäßen Anwendung (bzw. Verwendung) der Heyns-Oxidation (wie vorstehend beschrieben, vorzugsweise wie vorstehend als bevorzugt definiert) als Reaktionsgemisch erhalten bzw. eingesetzt (z.B. in einer kontinuierlichen Verfahrensgestaltung).

Die vorliegende Erfindung betrifft auch die Verwendung eines erfindungsgemäßen Gemisches (wie vorstehend beschrieben, vorzugsweise wie vorstehend als bevorzugt definiert) umfassend
- einen Katalysator, der Platin als Feststoff umfasst, bevorzugt Platin als Feststoff auf einem Trägermaterial umfasst, besonders bevorzugt Platin als Feststoff auf Kohlenstoff umfasst,
   sowie
   - eine oder mehrere Verbindungen ausgewählt aus der Gruppe bestehend aus
      (a) Verbindungen der Formel (II) 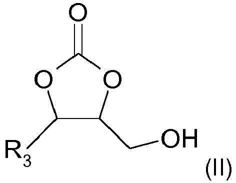 wobei R₃ ausgewählt ist aus H und geradkettigen, verzweigten oder cyclischen C₁₋₁₂-Alkylgruppen
         und optional eine oder mehrere Verbindungen ausgewählt aus der Gruppe bestehend aus
      (b) Verbindungen der Formel (III) 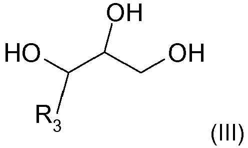 wobei R₃ ausgewählt ist aus H und geradkettigen, verzweigten oder cyclischen C₁₋₁₂-Alkylgruppen
   in einem Verfahren zur Herstellung einer Verbindung der Formel (I), vorzugsweise in einem erfindungsgemäßen Verfahren (wie vorstehend beschrieben, vorzugsweise wie vorstehend als bevorzugt definiert) zur Herstellung einer Verbindung der Formel (I). Besonders bevorzugt ist eine erfindungsgemäße Verwendung des zuvor beschriebenen erfindungsgemäßen Gemisches, wobei das erfindungsgemäße Gemisch sowohl eine oder mehrere (vorzugsweise eine, besonders bevorzugt eine Verbindung der Formel (II), wobei R₃ H bedeutet) Verbindungen der Formel (II) als auch eine oder mehrere (vorzugsweise eine, besonders bevorzugt eine Verbindung der Formel (III), wobei R₃ H bedeutet) Verbindungen der Formel (III) umfasst.

Nachfolgend wird die Erfindung durch Beispiele erläutert.

### Beispiele:

### 1. Genereller Versuchsaufbau:

In einem ersten Schritt wurde ein "Platin auf Kohle"-Katalysator in eine wässrige Glycerincarbonat-Lösung (Verbindung der Formel (II), wobei R₃ H bedeutet, in Wasser) gegeben (nachfolgend sind die Angaben zum Katalysator molar bezogen auf Glycerincarbonat).
In einem zweiten Schritt wurde die mit heterogenem Katalysator versetzte Lösung (nachfolgend Suspension genannt) auf die gewünschte Temperatur erwärmt und Sauerstoff (99,995%, Air Liquide) mit dem gewünschten Druck bzw. der gewünschten Flussrate (vgl. Tabelle 1) für die Dauer der Versuchsdurchführung mittels einer Fritte in die Suspension eingeleitet.
In einem dritten Schritt wurde (a) das Verhältnis von Verbindung der Formel (I) zu Verbindung der Formel (II) und (b) das Verhältnis von Verbindung der Formel (I) zu Verbindung der Formel (IV) mittels ¹H- und ¹³C-NMR zu verschiedenen Zeitpunkten bestimmt.
Weitere Details und Angaben zu den Versuchsparametern siehe nachfolgend unter Punkt 2 und Tabelle 1.

### 2. Spezifische Versuchsparameter und Ergebnisse:

Details und Versuchsparameter sind in der nachfolgenden Tabelle 1 zusammengefasst. Dabei bedeuten:
- "#" Beispielnummer
- "O₂[l/h]" Sauerstoffflussrate (bei offener Reaktionsführung; betrifft die Beispiele 1 bis 12)
- "Druck [bar]" Sauerstoffdruck im Reaktor (bei geschlossener Reaktionsführung; betrifft die Beispiele 13 bis 15)
- "GC [w/w%]" Glycerincarbonatkonzentration in Wasser
- "Pt [mol%]" eingesetzte Stoffmenge an geträgertem Platin bezogen auf die Gesamtstoffmenge Glycerincarbonat in der Suspension
- "Pt/C [w/w%]" Beladung des heterogenen Katalysators mit Platin
- "Austrag [%]"Rohausbeute bezogen auf 100% Umsatz, wobei sich Rohausbeute auf den Rückstand der Suspension nach Entfernen des Katalysators durch Filtration und Abdampfen leichter flüchtiger Bestandteile bezieht
- "(I):(II)" Stoffmengenverhältnis von Glycerinsäurecarbonat zu Glycerincarbonat (Maß für Umsatz und Selektivität)
- "(I):(IV)" Stoffmengenverhältnis von Glycerinsäurecarbonat zu Glycerinsäure (Maß für Selektivität und Ausmaß der Abbaureaktion).
Als Katalysatoren wurden Platin auf Kohle-Katalysatoren von Sigma Aldrich mit den folgenden Produktnummern eingesetzt:
Pt/C [w/w%] 10: Produktnummer: 80980 - Pt/C [w/w%] 5: Produktnummer: 80982

**Tabelle 1**

| # | O₂ [l/h] | Druck [bar] | T [°C] | t [h] | GC [w/w%] | Pt [mol%] | Pt/C [w/w %] | Austrag [%] | (I):(II) | (I):(IV) |
|---|---|---|---|---|---|---|---|---|---|---|
| 1 | 22,5 | - | 70 | 12 | 8,3 | 5 | 10 | 0 | - | - |
| 2 | 22,5 | - | 70 | 2 | 8,3 | 5 | 10 | - | 81 | 95 |
| 3 | 22,5 | - | 70 | 3 | 8,3 | 5 | 10 | - | 88 | 92 |
| 4 | 22,5 | - | 70 | 4 | 8,3 | 5 | 10 | 93 | 92 | 92 |
| 5 | 22,5 | - | 80 | 4 | 8,3 | 5 | 10 | 70 | 99 | 92 |
| 6 | 22,5 | - | 90 | 4 | 8,3 | 5 | 10 | 60 | 99 | 92 |
| 7 | 22,5 | - | 90 | 2 | 8,3 | 5 | 10 | - | 96 | 96 |
| 8 | 22,5 | - | 90 | 2 | 12 | 5 | 10 | - | 60 | 90 |
| 9 | 22,5 | - | 90 | 2 | 15,3 | 5 | 10 | - | 16 | 89 |
| 10 | 65,0 | - | 70 | 2 | 8,3 | 5 | 10 | - | 74 | 94 |
| 11 | 65,0 | - | 70 | 4 | 8,3 | 5 | 10 | - | 87 | 93 |
| 12 | 22,5 | - | 70 | 2 | 8,3 | 5 | 10 | - | 81 | 95 |
| 13 | - | 2 | 70 | 2 | 8,3 | 5 | 5 | 55 | 91 | 89 |
| 14 | - | 2 | 70 | 2 | 8,3 | 5 | 10 | 80 | 90 | 92 |
| 15 | - | 2 | 70 | 2 | 8,3 | 2,5 | 10 | 78 | 69 | 91 |

In den Beispielen 2, 3 und 7 bis 12 wurde der "Austrag" nicht bestimmt.
Beispiel 1 zeigt, dass nach einer Reaktionsdauer von 12 Stunden alle Reaktanden vollständig zu CO₂ zersetzt wurden. Eine besonders bevorzugte Reaktionsdauer liegt in einem Bereich von 2 bis 4 Stunden.
Beispiel 5 zeigt (verglichen mit den Beispielen 4 und 6), dass bei einer besonders bevorzugten Reaktionstemperatur von 80°C und einer bevorzugten Reaktionsdauer von 4 Stunden ein Optimum an Selektivität bei moderaten Austragseinbußen erreicht wurde.
Beispiel 7 zeigt (verglichen mit den Beispielen 8 und 9), dass eine Glycerincarbonatkonzentration von 8,3 w/w% zu sehr hohen "(I):(II)"- und "(I):(IV)"-Verhältnissen führt, verglichen mit Konzentrationen von 12 und 15,3 w/w% in den Beispielen 8 bzw. 9, bei ansonsten identischer Reaktionsdauer.
Die Beispiele 2, 10 und 11 zeigen, dass eine Erhöhung der Sauerstoffflussrate zu keiner nennenswerten Erhöhung der "(I):(II)" - und "(I):(IV)"-Verhältnisse führt (weder bei einer Reaktionsdauer von 2 noch von 4 Stunden).
Die Beispiele 12 und 14 zeigen hingegen, dass eine Erhöhung des Druckes im Reaktor (d.h. bei geschlossener Reaktionsführung) zu einer Erhöhung des "(I):(II)"-Verhältnisses führt.
Die vorstehend gezeigten Ergebnisse der durchgeführten Beispiele zeigen, dass die Beladung des heterogenen Katalysators mit Platin die Reaktionsparameter Reaktionsdauer und Reaktionstemperatur ("t" bzw. "T"), nicht aber die Qualität der Reaktion (abzulesen an den "(I):(II)"- und "(I):(IV)"-Verhältnissen) beeinflusst. So zeigen beispielsweise die Beispiele 13 und 14, dass die Reaktion mit einem Katalysator mit verringerter Beladung (Beispiel 13) zu ganz ähnlichen "(I):(II)"- und "(I):(IV)"-Verhältnissen führt, jedoch zu einem deutlich niedrigeren Austrag (bei identischer Reaktionsdauer).
Die Beispiele 14 und 15 zeigen, dass sich eine Verringerung der absoluten Platinmenge (vgl. Spalte Pt [mol%]) nachteilig auf das "(I):(II)"-Verhältnis auswirkt.

Gemäß Tabelle 1 stellen die Beispiele 4, 5 und 14 besonders bevorzugte Verfahrensdurchführungen dar.

In allen durchgeführten Oxidationsreaktionen wurde beobachtet, dass der pH-Wert des jeweiligen wässrigen Mediums nach 120 Minuten <0,5 war, ausgehend von einem anfänglichen pH-Wert im Bereich von 4 bis 5 (bei "t" = 0 Stunden und den in Tabelle 1 jeweils genannten Temperaturen). Der pH-Wert fiel somit in jedem der Beispiele um zumindest 3 pH-Einheiten. In keine der jeweiligen Suspensionen wurde eine Base oder ein Puffer hinzugegeben.

Der Verlauf der pH-Werte ist nachfolgend in Tabelle 2 an drei ausgewählten Beispielreaktionen detaillierter gezeigt. Die in Tabelle 2 gezeigte Beispielreaktion, die bei einer Temperatur von 70°C durchgeführt wurde, entspricht den Beispielen 10 und 11 in Tabelle 1. Die in Tabelle 2 gezeigte Beispielreaktion, die bei einer Temperatur von 80°C durchgeführt wurde, entspricht Beispiel 5, die Beispielreaktion, die bei 90°C durchgeführt wurde, entspricht Beispiel 6 in Tabelle 1.

**Tabelle 2:**

| 70°C | | | | 80°C | | | | 90°C | | | |
|---|---|---|---|---|---|---|---|---|---|---|---|
| Zeit [min] | (I):(II) | (I):(IV) | pH | Zeit [min] | (I):(II) | (I):(IV) | pH | Zeit [min] | (I):(II) | (I):(IV) | pH |
| 0 | 0 | 0 | 5,9 | 0 | 0 | 0 | 4,3 | 0 | 0 | 0 | 4,8 |
| 60 | 59 | 100 | 0.5 | 30 | 33 | 100 | 0.8 | 30 | 21 | 100 | 0.9 |
| 120 | 74 | 94 | 0,6 | 60 | 68 | 94 | 0.6 | 60 | 55 | 89 | 0.4 |
| 180 | 82 | 93 | 0,4 | 90 | 84 | 93 | 0.2 | 90 | 61 | 88 | 0.4 |
| 240 | 87 | 93 | 0,4 | 120 | 91 | 95 | 0.2 | 120 | 85 | 90 | 0.4 |
| 300 | 92 | 92 | 0,4 | 150 | 94 | 93 | 0.2 | 150 | 95 | 94 | 0.4 |
| 360 | 94 | 89 | 0,3 | 180 | 98 | 93 | 0.2 | 180 | 99 | 94 | 0.4 |
| 420 | 96 | 89 | 0,2 | 210 | 99 | 93 | 0.1 | 210 | 99 | 92 | 0.4 |
| 480 | 100 | 87 | 0,2 | 240 | 99 | 92 | 0.1 | 240 | 99 | 92 | 0.4 |

Des Weiteren wurde der Einfluss der pH-Stabilisierung untersucht. In Beispiel 16 wurde der pH-Wert während der Reaktion auf pH = 5,5 gepuffert, in Beispiel 17 wurde der pH-Wert nicht stabilisiert. Weitere Details und Versuchsparameter sind in der nachfolgenden Tabelle 3 zusammengefasst.

**Tabelle 3**

| # | O₂ [l/h] | Druck [bar] | T [°C] | t [h] | GC [w/w%] | Pt [mol%] | Pt/C [w/w%] | Pufferung [pH] | (I):(II) | (I):(IV) |
|---|---|---|---|---|---|---|---|---|---|---|
| 16 | 22,5 | - | 90 | 5 | 2,5 | 2,5 | 10 | 5,5 | 41 | 34 |
| 17 | 22,5 | - | 90 | 5 | 2,5 | 2,5 | 10 | - | 98 | 94 |

Der Vergleich zwischen den Beispielen 16 und 17 zeigt, dass viel höhere Umsätze und Selektivitäten erreicht werden können, wenn keine Pufferung erfolgt.

## Patentansprüche

1. Verfahren zur Herstellung einer Verbindung der Formel (I) 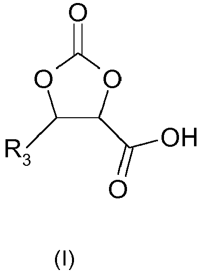 wobei R₃ ausgewählt ist aus H und geradkettigen, verzweigten oder cyclischen C₁₋₁₂-Alkylgruppen,
(a) mit folgenden Schritten:
Bereitstellen oder Herstellen einer Verbindung der Formel (II) 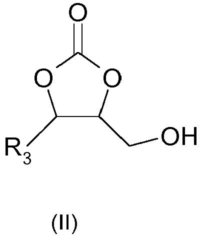
wobei R₃ die für Formel (I) gewählte Bedeutung hat, sowie
Platin-katalysiertes Oxidieren der Verbindung der Formel (II) mit gasförmigem Sauerstoff zu der Verbindung der Formel (I),
wobei der Schritt des Platin-katalysierten Oxidierens in wässrigem Medium erfolgt, zu Beginn des Schrittes des Platin-katalysierten Oxidierens ein pH-Wert von 7 oder kleiner 7 vorliegt und mit Bildung der Verbindung der Formel (I) zumindest um 2 pH-Einheiten fällt.

2. Verfahren nach Anspruch 1, wobei zum Platin-katalysierten Oxidieren ein Katalysator eingesetzt wird, der Platin als Feststoff umfasst, bevorzugt Platin als Feststoff auf einem Trägermaterial umfasst, besonders bevorzugt Platin als Feststoff auf Kohlenstoff umfasst.

3. Verfahren nach einem der vorangehenden Ansprüche, wobei R₃ Wasserstoff bedeutet.

4. Verfahren nach einem der vorangehenden Ansprüche, wobei zu Beginn des Schrittes des Platin-katalysierten Oxidierens im wässrigen Medium ein pH in einem Bereich von 4 bis 7 vorliegt, vorzugsweise ein pH in einem Bereich von 4 bis 6 vorliegt.

5. Verfahren nach Anspruch 4, wobei der Schritt des Platin-katalysierten Oxidierens so durchgeführt wird, dass mit Bildung der Verbindung der Formel (I) der pH zumindest um 3 pH-Einheiten, weiter bevorzugt zumindest um 4 pH-Einheiten, insbesondere bevorzugt um 6 pH-Einheiten fällt.

6. Verfahren nach einem der Ansprüche 4 bis 5, wobei der Schritt des Platin-katalysierten Oxidierens ohne Zugabe von Base oder Puffer erfolgt.

7. Verfahren nach einem der Ansprüche 4 bis 6, wobei der Schritt des Platin-katalysierten Oxidierens zumindest solange durchgeführt wird, bis ein pH-Wert von 2, vorzugsweise 1, besonders bevorzugt 0,5 unterschritten ist, wobei zu Beginn des Schrittes des Platin-katalysierten Oxidierens im wässrigen Medium ein pH-Wert von 7 oder kleiner 7 vorliegt, vorzugsweise ein pH in einem Bereich von 4 bis 7 vorliegt, vorzugsweise ein pH in einem Bereich von 4 bis 6 vorliegt.

8. Verfahren nach einem der vorangehenden Ansprüche, wobei zu Beginn des Schrittes des Platin-katalysierten Oxidierens das molare Verhältnis des eingesetzten Platins zu der Verbindung der Formel (II) größer ist als 2 : 100, vorzugsweise größer ist als 3 : 100.

9. Verfahren nach einem der vorangehenden Ansprüche, wobei der Schritt des Platin-katalysierten Oxidierens zumindest zeitweilig, vorzugsweise zeitlich überwiegend, bei einer Temperatur im Bereich von 50 bis 90°C, vorzugsweise im Bereich von 50 bis 82°C, besonders bevorzugt in einem Bereich von 68 bis 82°C erfolgt.

10. Verfahren nach einem der vorangehenden Ansprüche 4 bis 9, wobei der Schritt des Platin-katalysierten Oxidierens so durchgeführt wird, dass der gasförmige Sauerstoff in das wässrige Medium eingeleitet wird.

11. Anwendung der Heyns-Oxidation
(a) zur Oxidation einer Verbindung der Formel (II) 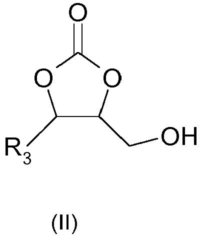
wobei R₃ ausgewählt ist aus H und geradkettigen, verzweigten oder cyclischen C₁₋₁₂-Alkylgruppen,
wobei die Heyns-Oxidation ohne Zugabe von Base oder Puffer erfolgt.

12. Gemisch umfassend
- einen Katalysator, der Platin als Feststoff umfasst, bevorzugt Platin als Feststoff auf einem Trägermaterial umfasst, besonders bevorzugt Platin als Feststoff auf Kohlenstoff umfasst,
sowie
- eine oder mehrere Verbindungen ausgewählt aus der Gruppe bestehend aus
(a) Verbindungen der Formel (II) 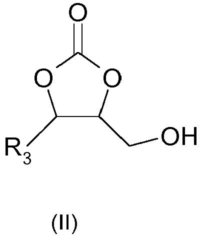 wobei R₃ ausgewählt ist aus H und geradkettigen, verzweigten oder cyclischen C₁₋₁₂-Alkylgruppen
und optional eine oder mehrere Verbindungen ausgewählt aus der Gruppe bestehend aus
(b) Verbindungen der Formel (III) 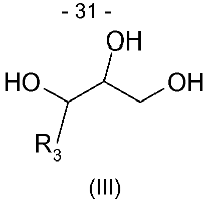 wobei R₃ ausgewählt ist aus H und geradkettigen, verzweigten oder cyclischen C₁₋₁₂-Alkylgruppen. wobei das Gemisch einen pH-Wert von 7 oder kleiner 7 aufweist.

## Claims

1. Method for preparing a compound of the formula (I) 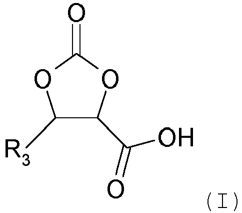 where R₃ is selected from H and straight-chain, branched or cyclic C₁₋₁₂-alkyl groups,
(a) with the following steps:
providing or preparing a compound of the formula (II) 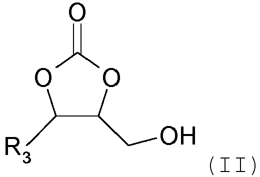
where R₃ has the definition selected for formula (I), and also
platinum-catalysed oxidation of the compound of the formula (II) with gaseous oxygen to give the compound of the formula (I),
wherein the platinum-catalysed oxidation step is carried out in an aqueous medium, at the start of the platinum-catalysed oxidation step the pH value is 7 or less than 7 and, on formation of the compound of the formula (I), decreases by at least 2 pH units.

2. Method according to Claim 1, wherein a catalyst is used for the platinum-catalysed oxidation comprising platinum as a solid, preferably comprising platinum as a solid on a support material, particularly preferably comprising platinum as a solid on carbon.

3. Method according to either of the preceding claims, where R₃ is hydrogen.

4. Method according to any of the preceding claims, wherein at the start of the platinum-catalysed oxidation step in the aqueous medium the pH is in a range from 4 to 7, preferably the pH is in a range from 4 to 6.

5. Method according to Claim 4, wherein the platinum-catalysed oxidation step is carried out such that, on formation of the compound of the formula (I), the pH decreases by at least 3 pH units, more preferably by at least 4 pH units, especially preferably by 6 pH units.

6. Method according to any of Claims 4 to 5, wherein the platinum-catalysed oxidation step is carried out without addition of base or buffer.

7. Method according to any of Claims 4 to 6, wherein the platinum-catalysed oxidation step is carried out at least until the pH value falls below 2, preferably below 1, particularly preferably below 0.5, wherein at the start of the platinum-catalysed oxidation step in the aqueous medium the pH value is 7 or less than 7, preferably the pH is in a range of 4 to 7, preferably the pH is in a range of 4 to 6.

8. Method according to any of the preceding claims, wherein at the start of the platinum-catalysed oxidation step the molar ratio of the platinum used to the compound of the formula (II) is greater than 2 : 100, preferably greater than 3 : 100.

9. Method according to any of the preceding claims, wherein the platinum-catalysed oxidation step is carried out at least intermittently, preferably for most of the time, at a temperature in the range of 50 to 90°C, preferably in the range of 50 to 82°C, particularly preferably in a range of 68 to 82°C.

10. Method according to any of the preceding Claims 4 to 9, wherein the platinum-catalysed oxidation step is carried out in such a way that the gaseous oxygen is introduced into the aqueous medium.

11. Use of the Heyns oxidation
(a) for oxidation of a compound of the formula (II) 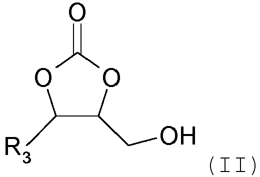
where R₃ is selected from H and straight-chain, branched or cyclic C₁₋₁₂-alkyl groups,
wherein the Heyns oxidation is carried out without addition of base or buffer.

12. Mixture comprising
- a catalyst comprising platinum as a solid, preferably comprising platinum as a solid on a support material, particularly preferably comprising platinum as a solid on carbon,
and also
- one or more compounds selected from the group consisting of
(a) compounds of the formula (II) 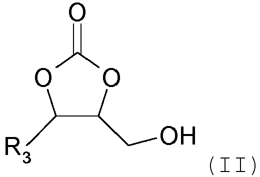 where R₃ is selected from H and straight-chain, branched or cyclic C₁₋₁₂-alkyl groups
and optionally one or more compounds selected from the group consisting of
(b) compounds of the formula (III) 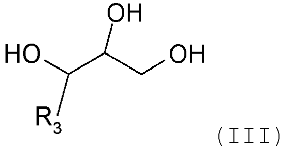 where R₃ is selected from H and straight-chain, branched or cyclic C₁₋₁₂-alkyl groups, where the mixture has a pH value of 7 or less than 7.

## Revendications

1. Procédé pour la préparation d'un composé de formule (I) 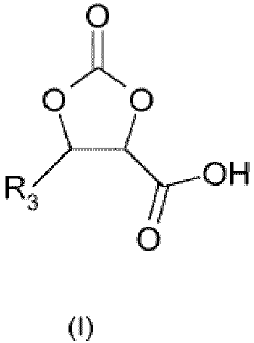 R₃ étant choisi parmi H et des groupes C₁₋₁₂-alkyle linéaires, ramifiés ou cycliques,
(a) selon les étapes suivantes :
mise à disposition ou préparation d'un composé de formule (II) 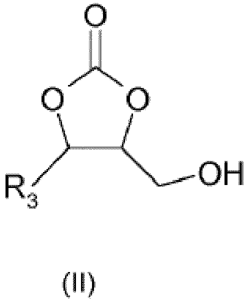
R₃ possédant la signification choisie pour la formule (I), ainsi que
oxydation catalysée au platine du composé de formule (II) avec de l'oxygène gazeux en composé de formule (I),
l'étape d'oxydation catalysée au platine étant réalisée en milieu aqueux, la valeur de pH étant de 7 ou inférieure à 7 au départ de l'étape d'oxydation catalysée au platine et, par la formation du composé de formule (I), chutant d'au moins 2 unités de pH.

2. Procédé selon la revendication 1, un catalyseur étant utilisé pour l'oxydation catalysée au platine, qui comprend du platine en tant que solide, préférablement qui comprend du platine en tant que solide sur un matériau de support, particulièrement préférablement qui comprend du platine en tant que solide sur du carbone.

3. Procédé selon l'une quelconque des revendications précédentes, R₃ signifiant hydrogène.

4. Procédé selon l'une quelconque des revendications précédentes, le pH étant dans une plage de 4 à 7 au début de l'étape d'oxydation catalysée au platine en milieu aqueux, de préférence le pH étant dans une plage de 4 à 6.

5. Procédé selon la revendication 4, l'étape d'oxydation catalysée au platine étant mise en œuvre de sorte que, par la formation du composé de formule (I), le pH chute d'au moins 3 unités de pH, plus préférablement d'au moins 4 unités de pH, particulièrement préférablement de 6 unités de pH.

6. Procédé selon l'une quelconque des revendications 4 et 5, l'étape d'oxydation catalysée au platine étant réalisée sans ajout d'une base ou d'un tampon.

7. Procédé selon l'une quelconque des revendications 4 à 6, l'étape d'oxydation catalysée au platine étant mise en œuvre du moins tant qu'une valeur de pH passe en dessous de 2, de préférence 1, particulièrement préférablement 0,5, la valeur de pH étant de 7 ou inférieure à 7 au départ de l'étape d'oxydation catalysée au platine en milieu aqueux, de préférence le pH étant dans une plage de 4 à 7, de préférence le pH étant dans une plage de 4 à 6.

8. Procédé selon l'une quelconque des revendications précédentes, le rapport molaire du platine utilisé au composé de formule (II) étant supérieur à 2:100, de préférence supérieur à 3:100, au début de l'étape d'oxydation catalysée au platine.

9. Procédé selon l'une quelconque des revendications précédentes, l'étape d'oxydation catalysée au platine étant réalisée au moins partiellement dans le temps, de préférence majoritairement dans le temps, à une température dans la plage de 50 à 90 °C, de préférence dans la plage de 50 à 82 °C, particulièrement préférablement dans une plage de 68 à 82 °C.

10. Procédé selon l'une quelconque des revendications précédentes 4 à 9, l'étape d'oxydation catalysée au platine étant réalisée de telle façon que l'oxygène gazeux est introduit dans le milieu aqueux.

11. Utilisation de l'oxydation de Heyns
(a) pour l'oxydation d'un composé de formule (II) 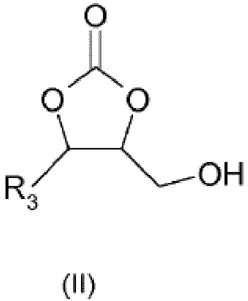
R₃ étant choisi parmi H et des groupes C₁₋₁₂-alkyle linéaires, ramifiés ou cycliques,
l'oxydation de Heyns étant réalisée sans ajout d'une base ou d'un tampon.

12. Mélange comprenant
- un catalyseur, qui comprend du platine en tant que solide, préférablement qui comprend du platine en tant que solide sur un matériau de support, particulièrement préférablement qui comprend du platine en tant que solide sur du carbone,
ainsi que
- un ou plusieurs composés choisis dans le groupe constitué par
(a) des composés de formule (II) 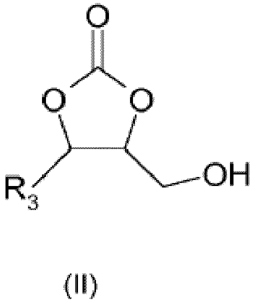
R₃ étant choisi parmi H et des groupes C₁₋₁₂-alkyle linéaires, ramifiés ou cycliques
et éventuellement un ou plusieurs composés choisis dans le groupe constitué par
(b) des composés de formule (III) 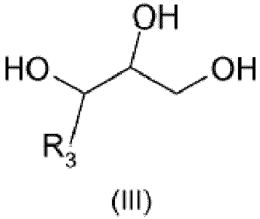 R₃ étant choisi parmi H et des groupes C₁₋₁₂-alkyle linéaires, ramifiés ou cycliques, le mélange présentant une valeur de pH de 7 ou inférieure à 7.
